# EUROPEAN PATENT SPECIFICATION

(11) **EP 2 142 071 B1**
(45) Date of publication and mention of the grant of the patent: **04.04.2018**
(21) Application number: 08746056.4
(22) Date of filing: 17.04.2008
(51) Int. Cl.: A61B 1/012, A61M 25/04, A61M 25/01, A61M 25/10, A61B 1/005, A61B 1/01, A61B 1/273

(54) **STEERABLE OVERTUBE**
STEUERBARES ÜBERROHR
ENVELOPPE TUBULAIRE ORIENTABLE

(30) Priority: 20.04.2007 US 925637 P
(43) Date of publication of application: 13.01.2010
(73) Proprietor: Cook Medical Technologies LLC, Bloomington, IN 47404 (US)
(72) Inventor: GIBBONS, William, S., Winston-salem, NC 27104 (US); SKERVEN, Gregory, J., Kernersville, NC 27284 (US)
(74) Representative: Garratt, Peter Douglas
(86) International application number: PCT/US2008/060572
(87) International publication number: WO 2008/140890

(56) References cited:
- EP-A- 0 343 094
- WO-A-93/01743
- WO-A-03/039354
- US-A1- 2003 229 296
- US-A1- 2005 272 975
- US-B1- 6 554 793

## Description

### TECHNICAL FIELD

The invention relates to a steerable overtube for use in introducing optical and other medical devices such as catheters and wire guides into a patient for performing minimally invasive medical procedures, and is particularly useful for performing procedures that have hereto been performed via an endoscope.

### BACKGROUND OF THE INVENTION

Endoscopes are routinely used to perform various medical procedures in areas of the body that are difficult to visualize or access, or that may otherwise require an open procedure to access. For example, endoscopes allow visual access to a target anatomy without the use of radioactive fluoroscopy. Endoscopes typically comprise an elongate shaft that is configured for introduction into the anatomy of a patient, for example, through the mouth, esophagus and stomach of a patient. A handle affixed to the proximal end of the shaft provides a control mechanism for manipulating the shape or direction of the distal end of the shaft, thereby allowing the endoscope to be "steered" through the patient's anatomy. The visual access capability of an endoscope is typically provided by image fibers and light carrying elements that extend through the shaft of the endoscope. Endoscopes also typically include a working channel through which other medical devices may be passed and directed to a target site within an internal body lumen or area of the anatomy. For example, catheters, wire guides and other types of elongated medical devices are frequently passed through the working channel of an endoscope to perform a diagnostic or medical procedure at a location near the distal end of the endoscope.

Currently available endoscopes have a number of significant drawbacks. One drawback is that the image and light carrying elements that extend through the shaft of the endoscope occupy a relatively large portion of the overall cross-sectional area of the shaft. And because the overall size and shape of the endoscope shaft is limited by the size and shape of the bodily lumen through which the endoscope shaft is configured to pass, the portion of the cross-sectional area of the shaft remaining for other components of the endoscope is severely limited. For example, the typical endoscope is limited to having only one moderately sized or two relatively small working channels. As a result, the number and size of medical devices that can be introduced through the endoscope is limited by these restrictions. However, there is a growing need to simultaneously introduce multiple medical devices, as well as larger medical devices, to the target site. For example, there is a growing need to introduce non-expandable stents and dilators having a relatively large diameter to treat strictures in bodily lumens such as the biliary and pancreatic ducts of a patient. These relatively large diameter stents and dilators, as well as other large diameter medical devices, cannot be introduced through the working channel of currently available endoscopes.

Another drawback is that currently available endoscopes are too large to pass through smaller lumens within the patient's anatomy. Thus, the visual access capability of the endoscope is limited to bodily lumens having a relatively large diameter, such as the esophagus, stomach, duodenum, colon, and large and small intestines. However, there is a growing need to visually access smaller bodily lumens such as the biliary tree. For example, there may be a need to visually observe a stricture or object, such as calculi, present within the common bile duct. Currently available endoscopes are too large to pass into the common duct.

On the other hand, many minimally invasive medical procedures typically performed through an endoscope rely primarily on fluoroscopy to guide and manipulate the various medical devices during the procedure. Thus, the visual access components, i.e., the image and light carrying elements incorporated into currently available endoscopes are often underutilized and/or unnecessary. However, because these visual access components are integral with the shaft of the endoscope, they cannot be removed so as to provide additional space for other components or devices. These visual access components are also expensive to manufacture, and add to the overall cost and complexity of the endoscope.

Still another drawback is that currently available endoscopes generally lack any mechanism for maintaining the shape of the endoscope shaft, or for securing the position of the endoscope shaft relative to the patient. For example, in many medical procedures performed in the biliary tree, medical devices such as wire guides and catheters must pass out through a port in the side of the endoscope, through the papilla and into the biliary tree. Thus, the portions of the medical devices extending out through the endoscope's distal port are typically disposed at a relatively large angle relative to the shaft of the endoscope. As a consequence, it may be difficult to apply the necessary leverage to advance these medical devices through the papilla and into the biliary tree. In addition, small and/or precise movements of these medical devices may be difficult because the shaft of the endoscope tends to move, as does the local anatomy, in response to the forces being applied to advance or manipulate the medical devices. Thus, there is a growing need for a more secure device delivery platform from which to advance or manipulate medical devices within the patient's anatomy. Reference is directed to US 2005/0272975 which discloses a structure according to the preamble of Claim 1.

### BRIEF SUMMARY OF THE INVENTION

Accordingly, the present invention provides a medical device having features that resolve or improve upon one or more of the above-described drawbacks.

According to the present invention, a steerable overtube is provided having an elongate shaft comprising an oversized accessory channel that is configured for the introduction and advancement of elongate medical devices having relatively large cross-sections. The distal end of the overtube shaft is bendable or deflectable in at least one direction, and is preferably bendable or deflectable in a plurality of directions. A control mechanism is operatively connected to the proximal end of the overtube shaft and provides a mechanism for controlling the bending or deflection of the distal end of the overtube shaft.

According to the present invention, the steerable overtube comprises a shape locking mechanism for temporarily maintaining the shape of the distal end of the overtube shaft. The shape locking mechanism may be integral with the control mechanism for controlling the bending or deflection of the distal end of the overtube shaft, or may be separately provided and operated.

According to another aspect of the present invention, the steerable overtube comprises multiple accessory channels, wherein at least one channel is oversized. The oversized channel may have a substantially larger cross-sectional area than the other accessory channels. One or more of the accessory channels may further include a mechanism for deflecting and/or securing an elongate medical device extending therethrough. For example, a movable elevator may be provided adjacent to the distal end of one or more of the channels, wherein movement of the elevator causes an elongate medical device to deflect along a pathway that diverges from the central axis of the overtube. The elevator may also be configured or moved so as to grasp the elongate medical device and prevent the medical device from moving longitudinally relative to the overtube. In certain embodiments of the present invention, a movable elevator is provided in each of a plurality of accessory channels of the steerable overtube. A mechanism for controlling movement of each of the elevators is also provided.

In the present invention, the steerable overtube comprises a fixation mechanism for securing the proximal end and/or distal end of the overtube against movement relative to the patient. In one embodiment, the fixation mechanism comprises a proximal securing device for securing the proximal end or a proximal portion of the overtube to the operating table or other stationary device. The proximal securing device prevents the proximal portion of the overtube (i.e., the portion that is extending out of the patient) from moving during the introduction, advancement, and/or manipulation of medical devices through the accessory channel(s) of the overtube.

The fixation mechanism comprises a distal anchoring device for temporarily securing and/or fixing the distal end of the overtube within the patient's anatomy. The distal anchoring device increases the leverage that can be applied to elongate medical devices as these devices are advanced beyond the distal end of the overtube. The distal anchoring device comprises a plurality of openings disposed about the perimeter of the distal end of the overtube shaft and operably connected to a suction source. Suction applied through these openings allows the distal end of the overtube to be temporarily affixed to a target area of the anatomy, for example, to the tissue surrounding the papilla along the inside wall of the duodenum. The distal anchoring device may also comprises a plurality of T-anchors or similar mechanical anchoring devices that can be deployed to allow the distal end of the overtube to be temporarily affixed to a target area of the anatomy, and/or one or more balloons disposed on the exterior surface of the overtube that may be inflated to engage the interior walls of the bodily lumen and thereby secure the overtube within the bodily lumen.

In yet another aspect of the present invention, a fiber optic elongate medical device is provided for use with the steerable overtube of the present invention. The fiber optic device may be configured to pass through an accessory channel of the steerable overtube, and may be extendable beyond the distal end of the overtube. The fiber optic device may also be removable from the accessory channel so as to allow the channel to be used for the introduction of other medical devices. Alternatively, the fiber optic device may be integrated into the steerable overtube of the present invention. However, it is desirable to minimize the size of the fiber optic device so as to maximize the cross-sectional area of the overtube that is available for other functions, such as accessory channels.

### BRIEF DESCRIPTION OF SEVERAL VIEWS OF THE DRAWINGS

Embodiments of the present invention will now be described by way of example with reference to the accompanying drawings, in which:
Figure 1 illustrates a perspective view of an exemplary embodiment of the steerable overtube of the present invention;
Figure 2 is a cross-sectional view of the shaft of the steerable overtube of the present invention taken along line 2-2 of Figure 1;
Figure 3 is a cross-sectional view of the handle assembly of the steerable overtube of the present invention taken along line 3-3 of Figure 1;
Figure 4 is a perspective view of an embodiment of the steerable overtube of the present invention comprising a proximal securing mechanism;
Figures 5 and 6 are perspective views of the distal end of an embodiment of the steerable overtube of the present invention comprising a distal anchoring device;
Figure 7 is a perspective view of the distal end of an embodiment of the steerable overtube of the present invention comprising an alternative distal anchoring device; and
Figure 8 is a perspective view of the distal end of an embodiment of the steerable overtube of the present invention comprising an elongate fiber optic device disposed therethrough.

### DETAILED DESCRIPTION

The invention is described with reference to the drawings in which like elements are referred to by like numerals. The relationship and functioning of the various elements of this invention are better understood by the following detailed description. However, the embodiments of this invention as described below are by way of example only, and the invention is not limited to the embodiments illustrated in the drawings. It should also be understood that the drawings are not to scale and in certain instances details have been omitted which are not necessary for an understanding of the present invention, such as conventional details of fabrication and assembly.

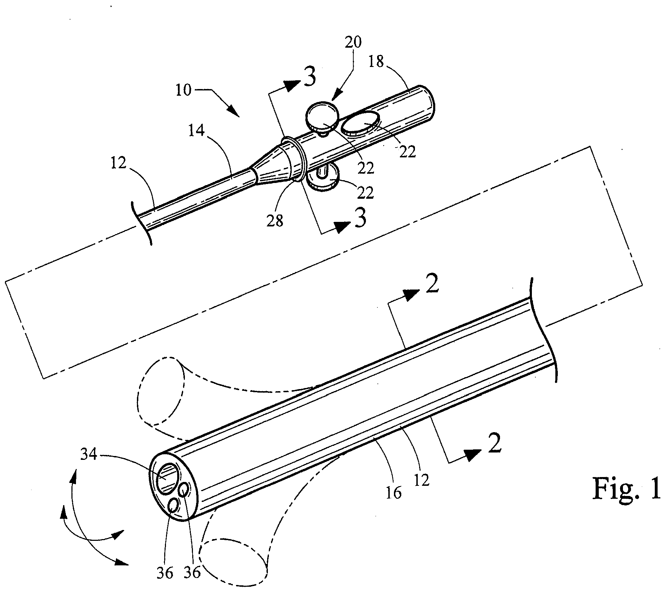

In general, Figure 1 illustrates an exemplary embodiment of the steerable overtube 10 of the present invention. The overtube 10 comprises an elongate shaft 12 having a proximal end 14 and a distal end 16. A handle assembly 18 is operatively connected to the proximal end 14 of the shaft 12, and includes control mechanism 20 for bending or deflecting the distal end 16 of the shaft 12. The distal end 16 of the shaft 12 is enlarged in Figure 1 to enhance the clarity of certain components described below. The shaft 12 may be manufactured from a variety of materials including shapeable plastic, nitinol, or combinations thereof that will allow the shaft 12 to bend or articulate, as will be discussed in greater detail below. The shaft 12, and particularly the distal end 16, may include radiopaque materials or markers that will permit the position of the shaft 12 within the patient to be observed using fluoroscopy.

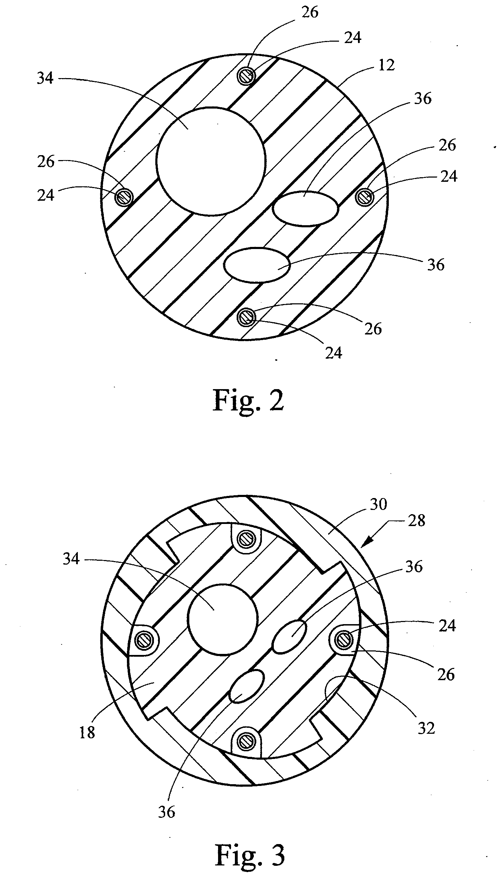

In the particular embodiment illustrated, the control mechanism 20 comprises a plurality of control knobs 22 rotatably connected to the handle assembly 18. Each control knob 22 is operably connected to a control wire 24 (see Figure 2) that extends through a lumen 26 in the shaft 12 to the distal end 16 thereof. As best seen in Figure 2, the overtube 10 comprises four control wires 24 disposed about the perimeter of the shaft 12 at spaced apart locations. The distal end of each control wire 24 is anchored to the shaft 12 so as to allow the transmission of tensile force from the control wire 24 to the distal end 16 of the shaft 12. The distal most portion of each lumen 26 is plugged or other wise filled in so as to prevent bodily fluids from entering therethrough.

Rotation of each control knob 22 causes movement of its respective control wire 24, which thereby tends to pull the distal end 16 of the shaft 12 in a proximal direction. Because of the resiliency of the shaft 12 and the eccentricity of the force applied by the control wire 24 to the shaft 12, this pulling action causes the distal end 16 to bend or deflect away from the central axis of the shaft 12, as illustrated in phantom lines on Figure 1. In the embodiment illustrated, the four control knobs 22 allow bending or deflection of the distal end 16 of the shaft in each of an upward, downward, left and right direction. However, it should be appreciated that fewer or more than four control knobs 22 (and control wires 24) may be utilized to achieve the desired deflection or bending of the distal end 16 of the shaft 12. For example, the overtube 10 may comprise three control knobs 22 (and three control wires 24) spaced about the perimeter of the shaft 12 at 120 degree increments. An exemplary control mechanism 20 that may be utilized with the steerable overtube 10 of the present invention is disclosed in U.S. Patent No. 5,325,845, entitled "Steerable Sheath For Use With Selected Removable Optical Catheter".

Other mechanisms for deflecting, bending or otherwise manipulating the shape or position of the distal end 16 of the overtube shaft 12 are also contemplated. For example, the distal end 16 of the shaft 12 may include magnetic or ferromagnetic materials that are capable of being manipulated by a magnetic force, such an externally generated electromagnetic field. The externally generated electromagnetic field may be applied through the patient and used to guide or manipulate the position of the overtube shaft 12.

The control mechanism 20 further comprises a shape locking mechanism 28 for temporarily maintaining the shape of the distal end 16 of the overtube shaft 12. In the embodiment illustrated in Figure 1, the shape locking mechanism 28 comprises a rotatable sleeve 30 that is operably connected to the proximal portion of the handle assembly 18. As best seen in Figure 3, the sleeve 30 may be rotated so as to engage with each of the control wires 24 and prevent movement of the control wires 24 relative to its respective lumen 26. More specifically, the sleeve 30 comprises cam surfaces 32 that are configured to engage each control wire 24 and clamp the control wire 24 against the inner surface of the lumen 26. Once the control wires 24 are clamped against longitudinal movement relative to lumens 26, then the shape of the distal end 16 of the shaft 12 will likewise be prevented from moving. Figure 3 illustrates the shape locking mechanism 28 in the unlocked configuration. Counter-clockwise movement of the sleeve 30 (relative to Figure 3) will cause the cam surfaces 32 to engage the control wires 24 and place the shape locking mechanism 28 in the locked configuration. In an exemplary procedure, the user may manipulate the control knobs 22 to effect movement of the distal end 16 of the shaft 12 upwardly and to the right. The user may then rotate sleeve 30 so as to lock the control wires 24, and consequently the distal end 16 of the shaft 12, into this specific shape.

Other shape locking mechanisms are also contemplated. For example, the shape locking mechanism may comprise a thumb screw attached to each of the control knobs 22 that may be tightened to prevent rotation thereof relative to the handle assembly 18. The thumb screws could also be configured to engage the control wires 24 in a similar fashion as the cam surfaces 32 of the above-described sleeve 30. Another type of shape locking mechanism is disclosed in U.S. Patent No. 6,942,613, entitled "Shape Lockable Apparatus and Method for Advancing an Instrument through Unsupported Anatomy".

The steerable overtube 10 of the present invention further comprises one or more accessory channels used for the introduction of various elongate medical devices. For example, in the embodiment illustrated in Figures 1-3, the overtube 10 comprises an oversized accessory channel 34 having a relative large cross-sectional area and configured to accommodate relatively large diameter medical devices therethrough. The overtube 10 further comprises a plurality of secondary accessory channels 36 for the introduction of smaller, more commonly sized medical devices. As best seen in Figure 2, the oversized accessory channel 34 has a cross-sectional area that is substantially larger than that of either of the secondary accessory channels 36. More importantly, the oversized accessory channel 34 has a cross-sectional area that is substantially larger than the accessory channels of commonly available endoscopes. In the embodiment illustrated, the oversized accessory channel 34 has a diameter of at least 3 mm, and preferably is 4 mm or larger in diameter. An oversized accessory channel 34 of this size will accommodate much larger medical devices, such as large stent delivery systems and dilators, than can be passed through the accessory channel of commonly available endoscopes.

Although one oversized and two smaller secondary accessory channels 34, 36 are shown, it should be appreciated that any number of channels having any combination of sizes can be utilized depending of the intended or anticipated types of medical procedures to be performed. For example, the overtube 10 of the present invention may utilize a single, very large oversized accessory channel 34. Or the overtube 10 may utilize two moderately oversized accessory channels 34 of similar size and shape. It should also be understood that the accessory channels, particularly the secondary channels 36, may comprise a non-circular cross-section. For example, a secondary accessory channel 36 utilized primarily for the introduction of contrast media may have a non-circular cross-section, which may allow additional area within the overtube shaft 12 for other components or a larger oversized accessory channel 34.

In the embodiment illustrated, the oversized and secondary accessory channels 34, 36 pass proximally through the handle assembly 18 and terminate at proximal openings in the proximal end thereof. However, one or more of the accessory channels 34, 36 may terminate in proximal ports located in the sidewall of the overtube shaft 12 a short distance distal of the handle assembly 18. Such a configuration may be advantageous because if prevents the accessory channels 34, 36 from interfering with the operating components in the handle assembly, such as the control mechanism 20 and the shape locking mechanism 28. It may also provide for a more ergonomic arrangement of these components, although the overall length of the shaft 12 may have to be increased.

One or more of the accessory channels 34, 36 may also include a mechanism for deflecting and/or securing an elongate medical device extending therethrough. For example, a movable elevator (not shown) may be provided adjacent to the distal end of one or more of the channels 34, 36, wherein movement of the elevator causes an elongate medical device to deflect along a pathway that diverges from the central axis of the distal end 16 of the overtube shaft 12. The elevator may also be configured or moved so as to grasp the elongate medical device and prevent the medical device from moving longitudinally relative to the overtube shaft 12. In certain embodiments of the present invention, a movable elevator is provided in each of a plurality of accessory channels 34, 36 of the steerable overtube 10. A mechanism for controlling movement of each of the elevators is also provided, and is preferably disposed on the handle assembly 18. Movable elevators and mechanisms for controlling the movement thereof are well known to those skilled in the art.

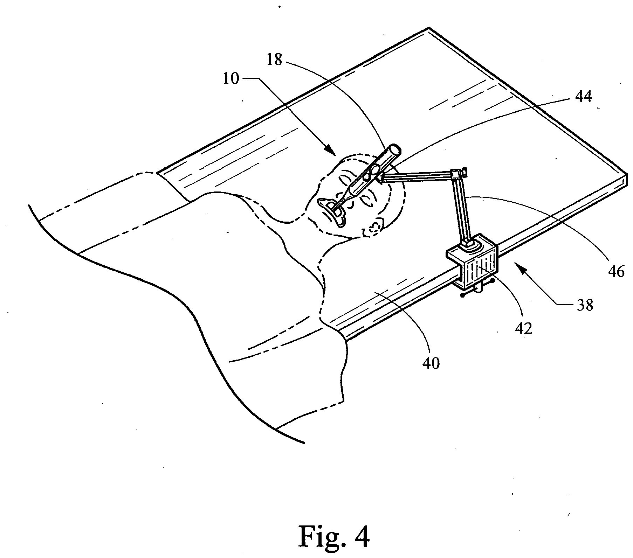

The steerable overtube 10 of the present invention further comprises a fixation mechanism for securing the proximal end 14 and/or distal end 16 of the overtube 10 against movement relative to the patient. In the embodiment illustrated in Figure 4, the fixation mechanism comprises a proximal securing device 38 for securing the proximal end (or proximal portion) of the overtube 10 to the operating table 40. The proximal securing device 38 prevents the proximal portion of the overtube 10 (i.e., the portion that is extending out of the patient) from moving during the introduction, advancement, and/or manipulation of medical devices through the accessory channels 34, 36 of the overtube 10. In the particular embodiment illustrated, the proximal securing device 38 comprises a base clamp 42 that is affixed to the operating table 40, a overtube clamp 44 that is affixed to the handle assembly 18 of the overtube, and an articulating arm 46 that is connected between the two clamps 42, 44. The articulating arm 46 preferably has a locking mechanism, such as frictional thumb screw, attached to each of the connections to the clamps 42, 44 and at any hinged joints therebetween so that the position of the arm 46 (and consequently the handle assembly 18) can be secured against movement relative to the operating table 40. This arrangement allows the user to release the overtube 10 once it has been properly positioned within the patient. In addition, this arrangement prevents the introduction, advancement, and manipulation of medical devices through the overtube 10 from inadvertently moving the overtube 10 relative to the patient.

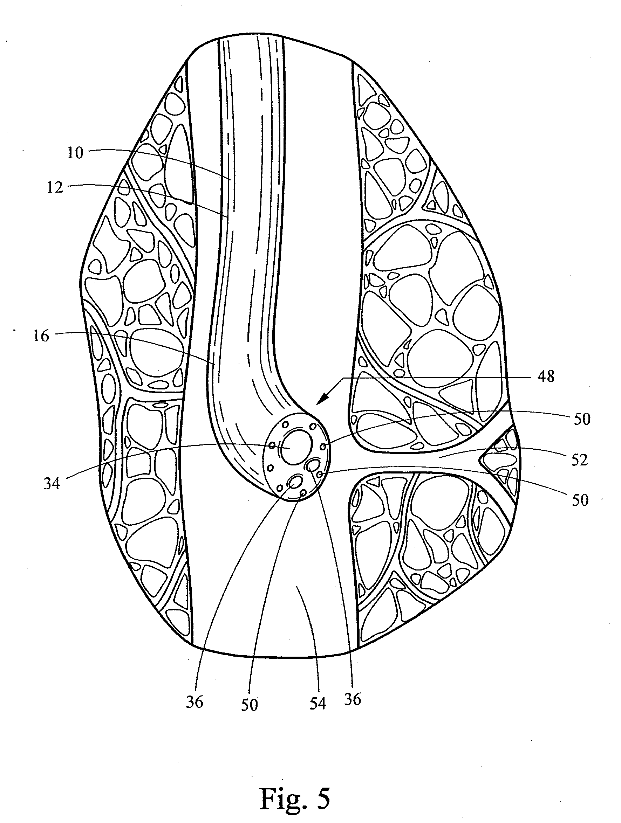

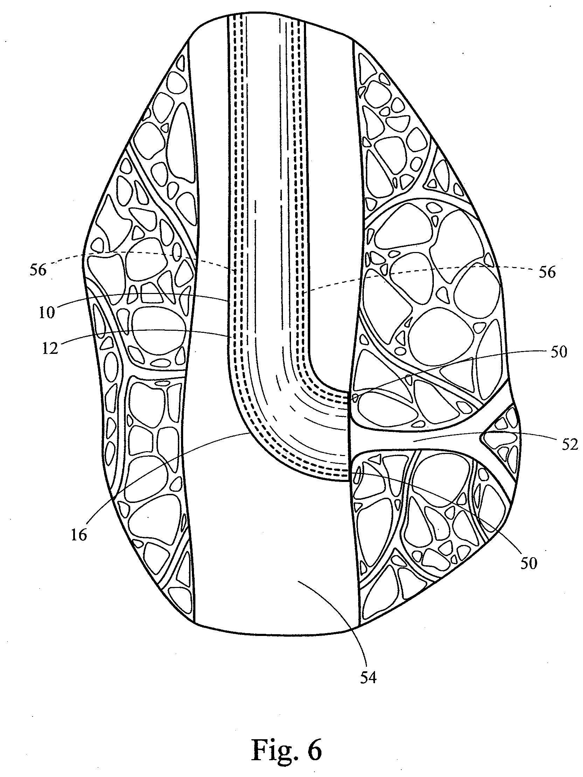

In particular the fixation mechanism comprises a distal anchoring device 48 for temporarily securing and/or fixing the distal end 16 of the overtube shaft 12 within the patient's anatomy. The distal anchoring device 16 increases the leverage that can be applied to the elongate medical devices as these devices are advanced beyond the distal end 16 of the overtube shaft 12. As illustrated in Figures 5 and 6, the distal anchoring device 48 comprises a plurality of openings 50 disposed about the perimeter of the distal end 16 of the overtube shaft 12 through which suction can be applied. Suction applied through these openings 50 allows the distal end 16 of the overtube shaft 12 to be temporarily affixed to a target area of the anatomy. As illustrated in Figure 6, suction applied through openings 50 allows the distal end 16 to be secured to the tissue surrounding the papilla 52 along the inside wall of the duodenum 54. In the particular embodiment illustrated, the openings 50 are in fluid communication with one or more lumens 56 (shown in phantom lines in Figure 6) extending through the shaft 12 of the overtube 10, which in turn are operably connected to a suction source (not shown). The suction source preferably is adjustable so as to allow the amount of suction applied to the openings 50 to be regulated to provide a secure attachment of the distal end 16 to the target tissue, but without causing damage to the tissue.

Securing the distal end 16 of the overtube shaft 12 to the patient's anatomy provides several advantages. One advantage is that the position of the distal end 16 of the overtube shaft 12 will be secured against movement during the introduction, advancement and manipulation of the medical devices therethrough. As a result, the likelihood of inadvertent movement of the distal end 16 of the overtube 10 relative to the target site is greatly reduced. The arrangement may also reduce trauma to the patient by focusing or centralizing the placement of medical devices as these devices are advanced beyond the distal end 16 of the shaft 12.

For example, once the distal end 16 of the overtube 10 is positioned in the duodenum 54 and secured to the tissue surrounding the papilla 52 (as shown in Figure 6), an ERCP catheter, sphincterotome or other elongate medical device (not shown) is advanced through the papilla 52 and into the common bile duct. The overtube 10 provides the necessary support or leverage to facilitate advancement of the elongate medical device through the papilla 52. In particular, the distal anchoring device 48, either alone or in combination with the proximal securing device 38 and/or the shape locking mechanism 28, guides and supports the elongate medical device as it is pushed through the papilla 52, which often provides substantial resistance to the passage of the device therethrough. Thereafter, the initial elongate medical device may be removed and replaced with a secondary elongate medical device, such as a stent delivery catheter, a lithotripsy basket, dilation balloon, biopsy forceps, extraction balloon or other interventional elongate medical device depending on the type of medical procedure being performed. The overtube 10, being secured in the manner illustrated in Figure 6, facilitates the introduction and advancement of these secondary medical devices through the papilla 52 and into the common bile duct without the need to reposition the overtube 10.

The distal anchoring device 48 may also comprise a plurality of T-anchors or similar mechanical anchoring devices that allow the distal end 16 of the overtube shaft 12 to be temporarily affixed to a target area of the anatomy. T-anchors and similar anchoring devices are often used to secure percutaneous devices such as feeding tubes to the stomach and abdominal walls of the patient. It is contemplated that these types of anchoring devices may be incorporated into the steerable overtube 10 of the present invention for the purpose describe above.

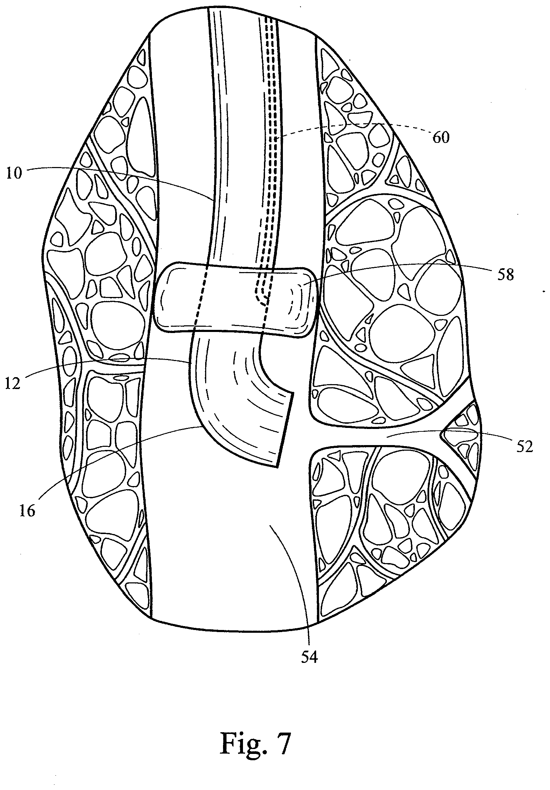

As illustrated in Figure 7, the distal anchoring device 48 may further comprise a balloon 58 disposed on the exterior surface of the overtube shaft 12 near the distal end 16 thereof. The balloon 58 is in fluid connection with an inflation lumen 60 (shown in phantom lines), which in turn is in fluid connection with a source of inflation fluid such as saline. The balloon 58 may be inflated so as to engage the interior walls of the bodily lumen and thereby secure the overtube 10 within the bodily lumen. In the embodiment illustrated, the balloon 58 is engaged with the interior wall of the duodenum 54. The balloon 58 preferably comprises a compliant material that will stretch to engage a sufficient portion of the wall of the bodily lumen so as to provide sufficient frictional contact there between.

In an alternative embodiment, a plurality of balloons may be disposed about the perimeter of the shaft 12 and separately connected to individual inflation sources, or to a single inflation source via regulating valves. A plurality of balloons permits the position of the overtube shaft 12 to be adjusted relative to the bodily lumen. For example, a balloon disposed along the side of the shaft 12 opposite the papilla 52 may be inflated to a lesser degree (or not at all) than the balloon on the side adjacent the papilla 52 so as to move the shaft 12 away from the papilla. In other words, a plurality of balloons may be selectively inflated to achieve minor adjustments in the position of the overtube shaft 12. Additional balloons may also be disposed along the length of the overtube shaft 12 at spaced apart locations to provide temporary anchoring of the overtube 10 to different parts of the patient's anatomy.

It should be understood that the steerable overtube 10 of the present invention may employ any combination of the above-described fixation mechanisms.

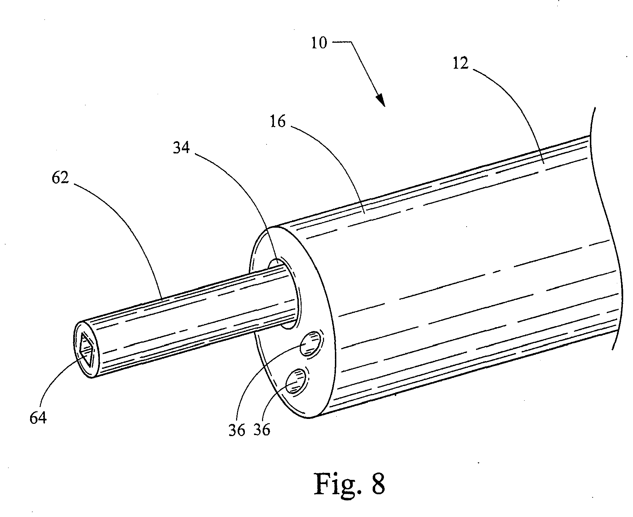

An elongate fiber optic device may be provided for use with the steerable overtube 10 of the present invention. As shown in Figure 8, the fiber optic device 62 may be configured to pass through the oversized accessory channel 34 of the steerable overtube 10, and may be extended beyond the distal end 16 of the overtube shaft 12. The fiber optic device 62 includes a lens 64 on the distal end thereof for transmitting images through the device to the user. The use of a separate fiber optic device 62 provides several advantages. First, the cross-sectional area of the fiber optic device 62 is relatively small, and much smaller than commonly available endoscopes. Thus, the fiber optic device 62 may be advanced into relatively small bodily lumens, such as the common bile and pancreatic ducts of the biliary tree. In addition, since the fiber optic device 62 is separate from the overtube 10, it may be introduced and/or removed at any point during the medical procedure, thereby making the oversized accessory channel 34 available for the introduction of other devices. This is particularly true since many minimally invasive medical procedures are often performed with fluoroscopy, thus making the need for integrated optics or other types of visual devices unnecessary. If the fiber optic device 62 has a sufficiently small cross-section, then it may also be introduced through one of the accessory channels 36. Alternatively, the fiber optic device 62 may be integrated into the steerable overtube 10 of the present invention. However, it is desirable to minimize the size of the fiber optic device 62 so as to maximize the cross-sectional area of the overtube 10 that is available for other functions, such as accessory channels 34, 36.

Although the steerable overtube 10 of the present invention has been described in connection with certain medical procedures performed in the biliary tree, it should be understood that it may be employed in many other types of medical procedures including colonoscopy, upper endoscopy, ultrasound endoscopy, and small bowel procedures. More specifically, the steerable overtube 10 of the present invention may be utilized in medical procedures that could only hereto be performed with endoscopes or endoscopic devices.

Any other undisclosed or incidental details of the construction or composition of the various elements of the disclosed embodiment of the present invention are not believed to be critical to the achievement of the advantages of the present invention, so long as the elements possess the attributes needed for them to perform as disclosed. The selection of these and other details of construction are believed to be well within the ability of one of even rudimentary skills in this area in view of the present disclosure. Illustrative embodiments of the present invention have been described in sufficient detail for the purpose of disclosing a practical, operative structure whereby the invention may be practiced advantageously. The designs described herein are intended to be exemplary only. The novel characteristics of the invention may be incorporated in other structural forms without departing from the scope of the invention.

Unless otherwise indicated, all ordinary words and terms used herein shall take their customary meaning as defined in The New Shorter Oxford English Dictionary, 1993 edition. All technical terms shall take on their customary meaning as established by the appropriate technical discipline utilized by those normally skilled in that particular art area. All medical terms shall take their meaning as defined by Stedman's Medical Dictionary, 27th edition.

## Claims

1. A steerable overtube (10) for use in introducing an elongate medical device into the biliary or pancreatic duct of a patient, the overtube comprising:
an elongate shaft (12) extending between a proximal end and a distal end, said distal end having a distal end face;
an oversized accessory channel (34) extending at least partially through the elongate shaft, the oversized accessory channel being configured for the passage of the elongate medical device therethrough;
a control mechanism (20) operatively connected to the proximal end of the shaft for controlling one of bending and deflecting of the distal end of the shaft;
a shape locking mechanism (28) for temporarily maintaining the shape of the distal end of the shaft; and
a fixation mechanism for securing the distal end of the shaft against movement relative to the patient;
wherein the fixation mechanism comprises a distal anchoring device (48) for temporarily securing the distal end of the overtube to a target tissue within the patient's anatomy;
**characterized in that** the distal anchoring device comprises a plurality of openings (50) disposed about a perimeter of and in the distal end face of the shaft through which suction can be applied to anchor the overtube to tissue surrounding the opening of the duct into the duodenum .

2. The steerable overtube according to claim 1 further comprising a proximal securing device for securing the proximal end of the shaft to a stationary object

3. The steerable overtube according to claim 2 wherein the stationary object is an operating table, and the proximal securing device comprises a first clamp secured to the operating table, a second clamp secured to proximal end of the shaft, and an articulating arm connected between the first and second clamps.

4. The steerable overtube according to claim 1 further comprising a secondary accessory channel extending at least partially through the elongate shaft, the secondary accessory channel being configured for the passage of a second elongate medical device therethrough.

5. The steerable overtube according to claim 1 wherein the oversized accessory channel has a diameter of at least 4 mm.

6. The steerable overtube according to claim 1 further comprising an elongate fiber optic device.

7. The steerable overtube according to claim 6 wherein the fiber optic device is movably disposed through the oversized accessory channel, and is extendable beyond the distal end of the shaft.

8. The steerable overtube according to claim 1 further comprising a handle assembly operably connected to the proximal end of the shaft, wherein the control mechanism and the shape locking mechanism are each operably connected to the handle assembly.

9. The steerable overtube according to claim 8 wherein the control mechanism comprises a plurality of rotatable knobs, each knob being operably connected to the distal end of the shaft via a control wire, wherein manipulation of one or more of the plurality of knobs causes the distal end of the shaft to deflect or bend away from a central axis of the shaft.

10. The steerable overtube according to claim 9 wherein the shape locking mechanism comprises a frictional device for temporarily securing one or more of the control wires against movement relative to the shaft or one or more of the plurality of knobs against movement relative to the handle assembly.

## Patentansprüche

1. Lenkbares Überrohr (10) zur Verwendung beim Einführen einer länglichen medizinischen Vorrichtung in den Gallen- oder Bauchspeicheldrüsengang eines Patienten, wobei das Überrohr Folgendes umfasst:
einen länglichen Schaft (12), der sich zwischen einem proximalen Ende und einem distalen Ende erstreckt, wobei das distale Ende eine distale Endfläche hat;
einen überdimensionierten Zubehörkanal (34), der sich mindestens teilweise durch den länglichen Schaft erstreckt, wobei der überdimensionierte Zubehörkanal für den Durchgang der länglichen medizinischen Vorrichtung dort hindurch konfiguriert ist;
einen Steuermechanismus (20), der mit dem proximalen Ende des Schafts wirkverbunden ist, um das Biegen oder Umlenken des distalen Endes des Schafts zu steuern;
einen Formverriegelungsmechanismus (28) zum vorübergehenden Beibehalten der Form des distalen Endes des Schafts; und
einen Fixierungsmechanismus zum Befestigen des distalen Endes des Schafts, so dass keine Bewegung bezüglich des Patienten erfolgt;
wobei der Fixierungsmechanismus eine distale Verankerungsvorrichtung (48) umfasst, um das distale Ende des Überrohrs vorübergehend an einem Zielgewebe in der Patientenanatomie zu befestigen;
**dadurch gekennzeichnet, dass** die distale Verankerungsvorrichtung eine Vielzahl von Öffnungen (50) umfasst, die um einen Umfang der distalen Endfläche des Schafts und in dieser angeordnet sind und durch die Saugwirkung angelegt werden kann, um das Überrohr an Gewebe zu verankern, das die Öffnung des Gangs in das Duodenum umgibt.

2. Lenkbares Überrohr nach Anspruch 1, ferner umfassend eine proximale Befestigungsvorrichtung zum Befestigen des proximalen Endes des Schafts an einem feststehenden Gegenstand.

3. Lenkbares Überrohr nach Anspruch 2, wobei der feststehende Gegenstand ein Operationstisch ist und die proximale Befestigungsvorrichtung eine erste am Operationstisch befestigte Klemme, eine zweite am proximalen Ende des Schafts befestigte Klemme und einen Gelenkarm umfasst, der zwischen der ersten und der zweiten Klemme verbunden ist.

4. Lenkbares Überrohr nach Anspruch 1, ferner umfassend einen sekundären Zubehörkanal, der sich mindestens teilweise durch den länglichen Schaft erstreckt, wobei der sekundäre Zubehörkanal für den Durchgang einer zweiten länglichen medizinischen Vorrichtung dort hindurch konfiguriert ist.

5. Lenkbares Überrohr nach Anspruch 1, wobei der überdimensionierte Zubehörkanal einen Durchmesser von mindestens 4 mm hat.

6. Lenkbares Überrohr nach Anspruch 1, ferner umfassend eine längliche faseroptische Vorrichtung.

7. Lenkbares Überrohr nach Anspruch 6, wobei die faseroptische Vorrichtung durch den überdimensionierten Zubehörkanal hindurch beweglich angeordnet und über das distale Ende des Schafts hinaus ausfahrbar ist.

8. Lenkbares Überrohr nach Anspruch 1, ferner umfassend eine Griffanordnung, die mit dem proximalen Ende des Schafts wirkverbunden ist, wobei der Steuermechanismus und der Formverriegelungsmechanismus jeweils mit der Griffanordnung wirkverbunden sind.

9. Lenkbares Überrohr nach Anspruch 8, wobei der Steuermechanismus eine Vielzahl von drehbaren Knöpfen umfasst, wobei jeder Knopf über einen Steuerdraht mit dem distalen Ende des Schafts wirkverbunden ist, wobei durch das Handhaben eines oder mehrerer der Vielzahl von Knöpfen veranlasst wird, dass das distale Ende des Schafts von einer mittleren Achse des Schafts weggelenkt oder weggebogen wird.

10. Lenkbares Überrohr nach Anspruch 9, wobei der Formverriegelungsmechanismus eine Reibvorrichtung umfasst, um einen oder mehrere der Steuerdrähte vorübergehend zu befestigen, so dass keine Bewegung bezüglich des Schafts erfolgt, oder einen oder mehrere der Vielzahl von Knöpfen vorübergehend zu befestigen, so dass keine Bewegung bezüglich der Griffanordnung erfolgt.

## Revendications

1. Gaine tubulaire orientable (10) destinée à une utilisation pour l'introduction d'un dispositif médical allongé dans le canal biliaire ou pancréatique d'un patient, la gaine tubulaire comprenant :
un corps allongé (12) s'étendant entre une extrémité proximale et une extrémité distale, ladite extrémité distale comportant une face d'extrémité distale ;
un conduit à accessoire surdimensionné (34) s'étendant au moins partiellement à travers le corps allongé, le conduit à accessoire surdimensionné étant configuré pour le passage du dispositif médical allongé à travers lui ;
un mécanisme de commande (20) relié de manière fonctionnelle à l'extrémité proximale du corps afin de commander une flexion ou une déviation de l'extrémité distale du corps ;
un mécanisme de verrouillage de forme (28) servant à maintenir temporairement la forme de l'extrémité distale du corps ; et
un mécanisme de fixation servant à assujettir l'extrémité distale du corps de façon à empêcher son déplacement par rapport au patient ;
le mécanisme de fixation comprenant un dispositif d'ancrage distal (48) servant à assujettir temporairement l'extrémité distale de la gaine tubulaire à un tissu cible au sein de l'anatomie du patient ;
**caractérisée en ce que** le dispositif d'ancrage distal comprend une pluralité d'ouvertures (50) disposées autour d'un périmètre de la face d'extrémité distale du corps et dans celle-ci, à travers lesquelles une aspiration peut être appliquée afin d'ancrer la gaine tubulaire au tissu entourant l'ouverture du canal débouchant dans le duodénum.

2. Gaine tubulaire orientable selon la revendication 1, comprenant en outre un dispositif d'assujettissement proximal servant à assujettir l'extrémité proximale du corps à un objet stationnaire.

3. Gaine tubulaire orientable selon la revendication 2, dans lequel l'objet stationnaire est une table d'opération, et le dispositif d'assujettissement proximal comprend un premier appareil de fixation par serrage assujetti à la table d'opération, un second appareil de fixation par serrage assujetti à l'extrémité proximale du corps et un bras d'articulation relié entre les premier et second appareils de fixation par serrage.

4. Gaine tubulaire orientable selon la revendication 1, comprenant en outre un conduit à accessoire secondaire s'étendant au moins partiellement à travers le corps allongé, le conduit à accessoire secondaire étant configuré pour le passage d'un second dispositif médical allongé à travers lui.

5. Gaine tubulaire orientable selon la revendication 1, dans lequel le conduit à accessoire surdimensionné présente un diamètre d'au moins 4 mm.

6. Gaine tubulaire orientable selon la revendication 1, comprenant en outre un dispositif à fibre optique allongé.

7. Gaine tubulaire orientable selon la revendication 6, dans lequel le dispositif à fibre optique est disposé de manière mobile à travers le conduit à accessoire surdimensionné, et peut être étendu au-delà de l'extrémité distale du corps.

8. Gaine tubulaire orientable selon la revendication 1, comprenant en outre un ensemble formant manche relié de manière fonctionnelle à l'extrémité proximale du corps, dans lequel le mécanisme de commande et le mécanisme de verrouillage de forme sont chacun reliés de manière fonctionnelle à l'ensemble formant manche.

9. Gaine tubulaire orientable selon la revendication 8, dans lequel le mécanisme de commande comprend une pluralité de boutons rotatifs, chaque bouton étant relié de manière fonctionnelle à l'extrémité distale du corps par le biais d'un fil de commande, dans lequel une manipulation d'un ou plusieurs boutons parmi la pluralité de boutons provoque une flexion ou une déviation de l'extrémité distale du corps par rapport à un axe médian du corps.

10. Gaine tubulaire orientable selon la revendication 9, dans lequel le mécanisme de verrouillage de forme comprend un dispositif de frottement servant à assujettir temporairement un ou plusieurs des fils de commande afin d'empêcher leur déplacement par rapport au corps ou un ou plusieurs boutons parmi la pluralité de boutons afin d'empêcher leur déplacement par rapport à l'ensemble formant manche.
